# EUROPÄISCHE PATENTSCHRIFT

(11) **EP 0 706 992 B1**
(45) Veröffentlichungstag und Bekanntmachung des Hinweises auf die Patenterteilung: **12.05.1999**
(21) Anmeldenummer: 95116092.8
(22) Anmeldetag: 12.10.1995
(51) Int. Cl.: C07C 69/50, C10M 105/46, C07C 69/34

(54) **Biologisch abbaubare, als Schmierstoff geeignete Oligoester**
Biodegradable oligoester useful as a lubricant
Oligoesters biodégradables utilisables comme lubrifiant

(30) Priorität: 15.10.1994 DE 4437007
(43) Veröffentlichungstag der Anmeldung: 17.04.1996
(73) Patentinhaber: Röhm GmbH, 64293 Darmstadt (DE)
(72) Erfinder: Hoppe, Dirk, Dr., D-48301 Nottuln (DE); Auschra, Clemens, Dr., D-55122 Mainz (DE)

(56) Entgegenhaltungen:
- US-A- 2 817 673
- DATABASE WPI Week 9308 Derwent Publications Ltd., London, GB; AN 93-061691 & JP-A-05 009 276 (TOYOBO KK) , 19.Januar 1993

## Beschreibung

Die Erfindung betrifft biologisch abbaubare, als Schmierstoff geeignete Oligoester aus einem tricyclischen Diol mit 8 bis 20 Kohlenstoffatomen, einer gesättigten, geradkettigen oder verzweigten Dicarbonsäure mit 4 bis 20 Kohlenstoffatomen und einem aliphatischen Alkohol mit 1 bis 30 Kohlenstoffatomen und die Verwendung der Oligoester. Die erfindungsgemäßen Oligoester weisen eine kinematische Viskosität von 50 bis 50 000 mm²/s bei 40 °C auf.

Synthetische Ester, wie zum Beispiel aliphatische und aromatische Dicarbonsäurediester, Dimersäureester und Pentaerythrittetraester, sowie natürliche Ester wie Rapsöl, werden in der Schmierstofftechnik in den unterschiedlichsten Bereichen eingesetzt. Sie zeichnen sich durch gute Schmierungseigenschaften aus, weisen, sofern es sich nicht um aromatische Ester handelt, einen hohen Viskositätsindex auf und sind in der Regel, im Gegensatz zu mineralölbasischen Produkten und sonstigen Kohlenwasserstoffen, wie zum Beispiel Polyalphaolefinen, biologisch gut abbaubar.

Nicht verfügbar sind bislang hochviskose Esteröle, die einen niedrigen Stockpunkt aufweisen, biologisch abbaubar sind und gute schmierstofftechnische Eigenschaften aufweisen. Die relativ hochviskosen Rapsöle und Dimersäureester neigen aufgrund der hier noch vorhandenen Doppelbindung zum Verharzen und sind sehr oxidationsempfindlich, so daß sie für viele Zwecke nicht einsetzbar sind.

US 2,817,673 beschreibt Ester mit Dihydroxymethylcyclododecan bzw. Cyclododecandicarbonsäure, basierend auf dem gleichen Diol-Baustein wie die Beispiele in dieser Anmeldung. Die in US 2,817,673 beschriebenen Oligoester sind aufgebaut aus Disäuren und Diolen. Im Gegensatz hierzu basieren die Oligoester aus dieser Anmeldung auf tricyclischem Diol, Dicarbonsäure und bestimmten aliphatischen Alkoholen. Für die in US 2,817,673 beschriebenen Ester wird als mögliche Anwendung u.a. sehr allgemein die Schmierstoffanwendung beschrieben. Eine besondere Eignung bezüglich biologischer Abbaubarkeit ist nicht erwähnt und ist aus US 2,817,673 auch nicht herleitbar.

Für den an Bedeutung zunehmenden Bereich der umweltfreundlichen Schmierstoffe besteht ein Bedarf an hochviskosen Grundflüssigkeiten (mit kinematischer Viskosität bie 40 °C von größer ca. 50 mm²/s), die biologisch leicht abbaubar sind (z.B. nach CEC L-33-A-94 zu > ca. 80 %). Zusätzlich müssen derartige Flüssigkeiten weitere wichtige schmierstofftechnische Eigenschatten erfüllen, wie z.B. niedriger Pour Point, gute Oxidations- und Hydrolysestabilität, Verträglichkeit mit anderen gängigen Gründölen und Additiven, Dichtungsverträglichkeit etc. Eine ausgewogenen Kombination dieser Eigenschatten wird von den am Markt derzeit verfügbaren Grundflüssigkeiten nur beschränkt erreicht. Sehr gut biologisch abbaubare Grundflüssigkeiten sind hauptsächlich nur in niedrigen Viskositätslagen verfügbar (KV 40 < 50 mm²/s). Hierbei handelt es sich meist um einfache Ester von aliphatischen Dicarbonsäuren (wie z.B. Ester der Adipinsäure) oder um Polyalphaolefine mit sehr niedrigem Molekulargewicht (PAO2). Für viele Anwendungen ist deren Viskosität jedoch zu gering und es müssen "unerwünschte" Verdicker zugegeben werden mit zum Teil schlechter biologischer Abbaubarkeit und/oder hohem Preis.

Das Molekulargewicht und damit die Viskosität der einfachen aliphatischen Diester kann durch Polykondensation erhöht werden (z.B. durch Polykondensation von Disäure mit Diolen. Infolge des höheren Molekulargewichts zeigen derartige Polyester meistens aber sehr viel schlechtere (= höhere) Pour Points, bzw. die Produkte liegen häufig bei Raumtemperatur sogar als Feststoffe vor oder sind schwerlöslich. Das Ausmaß der biologischen Abbaubarkeit kann nicht vorhergesagt werden. In vielen Fällen wird die Abbaubarkeit jedoch relativ schlecht sein, da diese erfahrungsgemäß mit zunehmendem Molekulargewicht schnell abnimmt. Insbesondere wurde bei cyclischen Verbindungen bislang immer von einer schlechteren biologischen Abbaubarkeit ausgegangen. Bei den gut abbaubaren Esterflüssigkeiten handelt es sich z.B. ausschließlich um nichtcyclische aliphatische Esterstrukturen wie z.B. Diisotridecyladipat.

Bei den in dieser Anmeldung beschriebenen speziellen Oligoestern aus Tricylischem Diol, Dicarbonsäure und aliphatischem Alkohol wurde entgegen der Erwartung gefunden, daß diese Produkte selbst bei realtiv hohen Viskositäten (KV40 > 100 mm²/s) noch gute biologische Abbaubarkeit besitzen und zudem unerwartet günstige (niedrige) Pour Points aufweisen.

Aufgabe der Erfindung war es deshalb, biologisch gut abbaubare, hochviskose Esteröle, die gute schmierstofftechnische Eigenschaften aufweisen, aufzufinden.

Es wurde nun überraschend gefunden, daß Oligoester aus einem tricylischen Diol, einer aliphatischen Dicarbonsäure und einem aliphatischen Alkohol als Endgruppe die obigen Anforderungen erfüllen.

Gegenstand der Erfindung sind daher Oligoester mit einer kinematischen Viskosität von 50 bis 50 000 mm²/s bei 40 °C, dadurch gekennzeichnet, daß sie aus einem tricyclischen Diol mit 10 bis 20 Kohlenstoffatomen, einer gesättigten, geradkettigen oder verzweigten Dicarbonsäure mit 4 bis 20 Kohlenstoffatomen und einem aliphatischen Alkohol mit 1 bis 30 Kohlenstoffatomen hergestellt werden. Weiterer Gegenstand der Erfindung ist die Verwendung der Oligoester. Das tricyclische Diol ist ein gesättigtes C₈ bis C₂₀-Diol. Das bevorzugte Diol ist ein Isomerengemisch des Dihydroximethyl-(5,2,1,0(^{2,6})tricylodecan, das im folgenden mit seinem Handelsnamen als "Dicidol" bezeichnet wird.

Die aliphatischen Dicarbonsäuren sind gesättigte, geradkettige oder verzweigte Dicarbonsäuren mit 4 bis 20 Kohlenstoffatomen. Bevorzugt sind Dicarbonsäuren mit 6 bis 18 Kohlenstoffatomen, besonders bevorzugt sind geradkettige Dicarbonsäuren mit 6 bis 18 Kohlenstoffatomen. Der aliphatische Alkohol ist ein geradkettiger oder verzweigter Alkohol mit 1 bis 30 Kohlenstoffatomen. Das molare Verhältnis von Dicarbonsäure zu tricyclischem Diol beträgt 1 : 0,3 bis 0,5 : 1. Bevorzugt sind Alkohole mit 2 bis 20 Kohlenstoffatomen. Die erfindungsgemäßen Oligoester können allein oder in Abmischung mit herkömmlichen Basisölen, wie zum Beispiel Dicarbonsäureestern, Mineralölen und Polyalphaolefinen, sowie geeigneten Additiven zur Formulierung einer breiten Palette von Schmierstoffen eingesetzt werden. Besonders geeignet sind die erfindungsmäßigen Oligoester zur Herstellung höherviskoser biologisch abbaubarer Schmierstoffe, wie zum Beispiel biologisch abbaubarer Hydraulik- und Kompressorenöle für den mobilen Außeneinsatz in Forst- und Landwirtschaft, im Bauwesen, im Bergbau sowie im Transportwesen.
Die folgenden Beispiele, sollen die Erfindung erläutern, ohne sie jedoch darauf einzuschränken.

### Beispiel 1

426 Teile Bis(2-ethylhexyl)sebacat und 98 Teile Dicidol werden in einem Dreihalskolben unter Rühren und Stickstoffspülung auf 160 °C erwärmt. Bei dieser Temperatur wird 1 Teil Tetrabutyltitanat zugegeben. Anschließend wird die Temperatur auf 180 °C erhöht und das entstehende 2-Ethylhexanol bei einem Druck von zunächst 300 mbar abdestilliert. Der Druck wird danach bis auf 0,2 mbar reduziert, um den Alkohol vollständig zu entfernen. Nach Abkühlen auf 100 °C werden 20 Teile Wasser hinzugegeben und nach einstündigem Rühren bei dieser Temperatur das Titanoxidhydrat durch Filtration entfernt. Die Ausbeute ist nahezu quantitativ. Das Produkt weist die folgenden Eigenschaften auf: (KV: Kinematische Viskosität)

| | | |
|---|---|---|
| KV (40 °C) | 146,2 mm²/s | DIN 51 562 |
| KV (100 °C) | 20,3 mm²/s | DIN 51 562 |
| Viskositätsindex | 161,1 | DIN ISO 2909 |
| Pour Point | -45 °C | DIN ISO 3016 |
| FZG Gutlaststufe | 6 % | DIN 51 354 |
| Biologische Abbaubarkeit | 88 % | CEC-L-33-T-82 |

### Beispiel 2

426 Teile Bis(2-ethylhexyl)sebacat und 108 Teile Dicidol werden in einem Dreihalskolben unter Rühren und Stickstoffspülung auf 160 °C erwärmt. Bei dieser Temperatur wird 1 Teil Tetrabutyltitanat zugegeben. Anschließend wird die Temperatur auf 190 °C erhöht und das entstehende 2-Ethylhexanol bei einem Druck von zunächst 300 mbar abdestilliert. Der Druck wird danach bis auf 0,2 mbar reduziert, um den Alkohol vollständig zu entfernen. Nach Abkühlen auf 100 °C werden 20 Teile Wasser hinzugegeben und nach einstündigem Rühren bei dieser Temperatur das Titanoxidhydrat durch Filtration entfernt. Die Ausbeute ist nahezu quantitativ. Das Produkt weist die folgenden Eigenschaften auf: (KV: Kinematische Viskosität)

| | | |
|---|---|---|
| KV (40 °C) | 228,3 mm²/s | DIN 51 562 |
| KV (100 °C) | 27,9 mm²/s | DIN 51 562 |
| Viskositätsindex | 159,0 | DIN ISO 2909 |
| Pour Point | -45 °C | DIN ISO 3016 |
| FZG Gutlaststufe | 6 % | DIN 51 354 |
| Biologische Abbaubarkeit | 91 % | CEC-L-33-T-82 |

### Beispiel 3

426 Teile Bis(2-ethylhexyl)sebacat und 138 Teile Dicidol werden in einem Dreihalskolben unter Rühren und Stickstoffspülung auf 160 °C erwärmt. Bei dieser Temperatur wird 1 Teil Tetrabutyltitanat zugegeben. Anschließend wird die Temperatur auf 190 °C erhöht und das entstehende 2-Ethylhexanol bei einem Druck von zunächst 300 mbar abdestilliert. Der Druck wird danach bis auf 0,2 mbar reduziert, um den Alkohol vollständig zu entfernen. Nach Abkühlen auf 100 °C werden 20 Teile Wasser hinzugegeben und nach einstündigem Rühren bei dieser Temperatur das Titanoxidhydrat durch Filtration entfernt. Die Ausbeute ist nahezu quantitativ. Das Produkt weist die folgenden Eigenschaften auf: (KV: Kinematische Viskosität)

| | | |
|---|---|---|
| KV (40 °C) | 966,3 mm²/s | DIN 51 562 |
| KV (100 °C) | 81,5 mm²/s | DIN 51 562 |
| Viskositätsindex | 164,0 | DIN ISO 2909 |
| Pour Point | -45 °C | DIN ISO 3016 |
| FZG Gutlaststufe | 5 % | DIN 51 354 |
| Biologische Abbaubarkeit | 80 % | CEC-L-33-T-82 |

### Beispiel 4

426 Teile Bis(2-ethylhexyl)sebacat und 108 Teile Dicidol werden in einem Dreihalskolben unter Rühren und Stickstoffspülung auf 160 °C erwärmt. Bei dieser Temperatur wird 1 Teil Tetrabutyltitanat zugegeben. Anschließend wird die Temperatur auf 190 °C erhöht und das entstehende 2-Ethylhexanol bei einem Druck von zunächst 300 mbar abdestilliert. Der Druck wird danach bis auf 0,2 mbar reduziert, um den Alkohol vollständig zu entfernen. Nach Abkühlen auf 100 °C werden 20 Teile Wasser hinzugegeben und nach einstündigem Rühren bei dieser Temperatur das Titanoxidhydrat durch Filtration entfernt. Die Ausbeute ist nahezu quantitativ. Das Produkt weist die folgenden Eigenschaften auf: (KV: Kinematische Viskosität)

| | | |
|---|---|---|
| KV (40 °C) | 245,2 mm²/s | DIN 51 562 |
| KV (100 °C) | 28,3 mm²/s | DIN 51 562 |
| Viskositätsindex | 164,0 | DIN ISO 2909 |
| Pour Point | -45 °C | DIN ISO 3016 |
| FZG Gutlaststufe | 6 % | DIN 51 354 |
| Biologische Abbaubarkeit | 92 % | CEC-L-33-T-82 |

## Patentansprüche

1. Oligoester mit einer kinematischen Viskosität von 50 bis 50 000 mm²/s bei 40 °C,
dadurch gekennzeichnet,
daß sie aus einem tricyclischen Diol mit 8 bis 20 Kohlenstoffatomen, einer gesättigten, geradkettigen oder verzweigten Dicarbonsäure mit 4 bis 20 Kohlenstoffatomen und einem aliphatischen Alkohol mit 1 bis 30 Kohlenstoffatomen hergestellt werden.

2. Oligoester gemäß Anspruch 1,
dadurch gekennzeichnet,
daß das tricyclische Diol ein Dihydroximethyl (5,2,1,0^{(2,6)})tricyclodecan ist.

3. Oligoester gemäß Anspruch 1,
dadurch gekennzeichnet,
daß die Dicarbonsäure 6 bis 18 Kohlenstoffatome enthält.

4. Oligoester gemäß Anspruch 1,
dadurch gekennzeichnet,
daß der aliphatische Alkohol 2 bis 20 Kohlenstoffatome enthält.

5. Oligoester gemäß Anspruch 1,
dadurch gekennzeichnet,
daß das molare Verhältnis von Disäure zu tricyclischem Diol von 1 : 0,3 bis 0,5 : 1 beträgt.

6. Verwendung der Oligoester gemäß Anspruch 1 für sich allein oder in Abmischung mit bekannten Ölen als Grundöl für Schmierstoffe.

7. Verwendung der Oligoester gemäß Anspruch 1 allein oder in Abmischung mit bekannten biologisch abbaubaren Ölen als Grundöl für biologisch abbaubare Schmierstoffe.

8. Verwendung der Oligoester gemäß Anspruch 1 allein oder in Abmischung mit bekannten biologisch abbaubaren Ölen als Grundöl für biologisch abbaubare Hydraulik- und Kompressorenölen für Maschinen im mobilen Außeneinsatz.

## Claims

1. Oligoesters with a kinematic viscosity of 50 to 50 000 mm²/s at 40°C, characterised in that they can be produced from a tricyclic diol with 8 to 20 carbon atoms, a saturated, straight-chained or branched dicarbonic acid with 4 to 20 carbon atoms and an aliphatic alcohol with 1 to 30 carbon atoms.

2. Oligoesters according to claim 1, characterised in that the tricyclic diol is a dihydroximethyl (5,2,1,0^{(2,6)})tricyclodecane.

3. Oligoesters according to claim 1, characterised in that the dicarbonic acid contains 6 to 18 carbon atoms.

4. Oligoesters according to claim 1, characterised in that the aliphatic alcohol contains 2 to 20 carbon atoms.

5. Oligoesters according to claim 1, characterised in that the molar ratio of diacid to tricyclic diol is from 1:0.3 to 0.5:1.

6. Use of the oligoesters according to claim 1 alone or mixed with known oils as a base oil for lubricants.

7. Use of the oligoesters according to claim 1 alone or mixed with known biologically-degradable oils as a base oil for biologically-degradable lubricants.

8. Use of the oligoesters according to claim 1 alone or mixed with known biologically-degradable oils as a base oil for biologically-degradable hydraulic and compressor oils for machines in mobile use outdoors.

## Revendications

1. Oligoesters ayant une viscosité cinématique allant de 50 à 50.000 mm²/s à 40°C,
caractérisé en ce qu'
ils sont préparés à partir d'une diol tricyclique ayant de 8 à 20 atomes de carbone, un acide dicarboxylique saturé, à chaîne droite ou ramifié ayant de 4 à 20 atomes de carbone et d'un alcool aliphatique ayant de 1 à 30 atomes de carbone.

2. Oligoesters selon la revendication 1,
caractérisés en ce que
le diol tricyclique est un dihydroxyméthyl (5,2, 1,0^{(2,6)})tricyclodécane.

3. Oligoesters selon la revendication 1,
caractérisés en ce que
l'acide dicarboxylique renferme de 6 à 18 atomes de carbone.

4. Oligoesters selon la revendication 1,
caractérisés en ce que
l'alcool aliphatique renferme de 2 à 20 atomes de carbone.

5. Oligoesters selon la revendication 1,
caractérisés en ce que
le rapport molaire de l'acide de dicarboxylique au diol tricyclique s'élève à de 1:0,3 à 0,5:1.

6. Utilisation des oligoesters selon la revendication 1, seule ou en mélange avec des huiles communes, en tant qu'huile de base pour des lubrifiants.

7. Utilisation des oligoesters selon la revendication 1, seule ou en mélange avec des huiles biodégradables comme en tant qu'huile de base pour lubrifiants dégradables biologiquement.

8. Utilisation des oligoesters selon la revendication 1, seule ou en mélange avec des huiles connues biologiquement dégradables en tant qu'huile de base pour des huiles hydrauliques et de compresseurs biologiquement dégradables pour des machines en utilisation extérieure mobile.
